# FASCICULE DE BREVET EUROPEEN

(11) **EP 1 451 574 B1**
(45) Date de publication et mention de la délivrance du brevet: **16.01.2019**
(21) Numéro de dépôt: 02796884.1
(22) Date de dépôt: 28.11.2002
(51) Int. Cl.: G01N 33/18, G01N 21/33

(54) **PROCEDE DE MESURE DE LA CONCENTRATION DES DERIVES CHLORES DE L'ACIDE ISOCYANURIQUE DANS L'EAU**
VERFAHREN ZUR BESTIMMUNG DER KONZENTRATION CHLORIERTER ISOCYANURSÄUREDERIVATE IM WASSER
METHOD FOR MEASURING CONCENTRATION OF CHLOROISOCYANURIC ACID DERIVATIVES IN WATER

(30) Priorité: 30.11.2001 FR 0115495
(43) Date de publication de la demande: 01.09.2004
(73) Titulaire: Syclope Electronique, 64230 Lescar (FR)
(72) Inventeur: Breton, Georges, 64121 Serres-Castet (FR); Fichot, Philippe Résidence Jardins d'Arcins, 33130 Bégles (FR)
(74) Mandataire: Intès, Didier Gérard André
(86) Numéro de dépôt international: PCT/FR2002/004082
(87) Numéro de publication internationale: WO 2003/046549

(56) Documents cités:
- US-A- 4 855 239
- US-A- 5 230 785
- US-A- 5 661 037
- J.GARDINER: "Chloroisocyanurates in the treatment of swimming pool water" WATER RESEARCH, vol. 7, 1973, pages 823-833, XP008006205
- OBRIEN J.E.; MORRIS J.C.; BUTLER J.N.: "Equilibria in aqueous solutions of chlorinated isocyanurate", AMERICAN CHEMICAL SOCIETY SYMPOSIUM 1973 ON CHEMISTRY OF WATER SUPPLY, TREATMENT & DISTRIBUTION, 1 January 1974 (1974-01-01), pages 333-358, XP008086657,

## Description

La présente invention se rapporte à un procédé et un dispositif de mesure de la concentration des dérivés chlorés de l'acide isocyanurique en équilibre dans une solution aqueuse.

Un domaine d'application envisagé est notamment, mais non exclusivement, celui du contrôle de la qualité des eaux de piscine, dans lesquelles on introduit des chlorocyanurates susceptibles de produire de l'acide hypochloreux, HOCl, lequel est un puissant désinfectant.

Cependant, pour que le processus de chloration de l'eau soit efficace, il est nécessaire que la concentration en dérivés chlorés de l'acide isocyanurique soit inférieure à un seuil déterminé, qu'il est nécessaire de mesurer pour pouvoir, le cas échéant, l'ajuster. L'acide isocyanurique est généralement symbolisé par le symbole H₃Cy dont les formes ioniques principales dans l'eau de piscine, par exemple, où le pH est situé entre 6,5 et 7,5, sont H₂Cy⁻ et HClCy⁻. Le document US 5,230,785 décrit un dispositif de mesure selon le préambule de la revendication 6.

Des procédés connus de contrôle du taux de chloroisocyanurates, ou dérivés chlorés de l'acide isocyanurique, sont mis en oeuvre avec des réactifs particuliers qui provoquent des troubles plus ou moins intenses en fonction de la concentration en chloroisocyanurates. Ainsi, on prélève un échantillon de l'eau à analyser dans lequel on introduit ledit réactif et on mesure qualitativement la présence de chloroisocyanurates par l'observation du trouble ou quantitativement au moyen d'une méthode photométrique.

Ces procédés de mesure, présentent l'inconvénient d'être peu précis et de donner un résultat de mesure avec une précision de l'ordre de 30% dans le cas le plus favorable. En outre, ils ne sont pas sensibles aux variations de pH qui modifient considérablement la concentration des différents dérivés chlorés de l'acide isocyanurique et leur mise en oeuvre au moyen d'un système automatique de contrôle est mal aisée.

Comme autre procédé, il est également connu, pour mesurer la concentration des dérivés chlorés de l'acide isocyanurique en équilibre dans une solution aqueuse, de déterminer les constantes d'absorption dans l'ultraviolet de ces différents composés; déterminer l'acidité de la solution aqueuse pour fournir des valeurs relatives des concentrations de ces dérivés ; mesurer l'absorbance d'un faisceau de rayonnement ; déterminer en fonction de celle-ci des valeurs de concentration des dérivés. Un tel procédé est détaillé par le document « Equilibria in aqueous solutions of chlorinated isocyanurate » de l'American Chemical Society Symposium 1973 on Chemistry of Water Supply, Treatment and Distribution, 1 Janvier 1974, pages 333-347, XP008086657, de J.E.O'Brien, J.C.Morris, J.N.Butler.

Un problème qui se pose et que vise à résoudre la présente invention est alors de mettre en oeuvre un procédé de mesure de la concentration en dérivés chlorés de l'acide isocyanurique en équilibre dans une solution aqueuse qui non seulement permette d'obtenir une mesure avec une meilleure précision mais aussi qui soit susceptible d'être mis en oeuvre par un dispositif automatique fournissant des mesures avec une grande fréquence.

A cet effet, un premier objet de la présente invention est de proposer un procédé de mesure de la concentration des dérivés chlorés de l'acide isocyanurique comprenant les étapes suivantes : on détermine les coefficients d'absorption de chacun des premiers dérivés chlorés de l'acide isocyanurique dans ladite solution aqueuse susceptible d'absorber un faisceau de rayonnement d'une longueur d'onde comprise entre 210 et 220 nanomètres ; on détermine l'acidité de ladite solution aqueuse pour fournir des valeurs relatives des concentrations des différents dérivés chlorés de l'acide isocyanurique en équilibre dans ladite solution aqueuse ; on mesure une première valeur de l'absorbance d'un premier faisceau de rayonnement traversant ladite solution aqueuse, ledit premier faisceau de rayonnement ayant une longueur d'onde comprise entre 210 et 220 nanomètres, de façon à déterminer au moins la valeur de l'absorbance totale de premiers dérivés chlorés de l'acide isocyanurique ; et, on détermine en fonction de ladite première valeur de l'absorbance dudit premier faisceau de rayonnement traversant ladite solution aqueuse, desdits coefficients d'absorption desdits premiers dérivés chlorés de l'acide isocyanurique et desdites valeurs relatives des concentrations des différents dérivés chlorés de l'acide isocyanurique, des premières valeurs absolues des concentrations des différents dérivés chlorés de l'acide isocyanurique, lesdites premières valeurs absolues correspondant sensiblement aux valeurs absolues réelles des concentrations des différents dérivés chlorés de l'acide isocyanurique en équilibre dans ladite solution aqueuse.

Ainsi, une caractéristique de l'invention réside dans le mode de détection et de quantification des dérivés chlorés de l'acide isocyanurique, dont certains, lesdits premiers, sont sensibles au rayonnement électromagnétique situé dans le domaine de l'ultraviolet et plus particulièrement entre 210 et 220 nanomètres. C'est notamment le cas des formes ionisées H₂Cy⁻ et HClCy⁻. De la sorte, en tenant compte des valeurs du pH qui déterminent la proportion des différentes formes ionisées des dérivés chlorés de l'acide isocyanurique les unes par rapport aux autres et en négligeant les formes ionisées fortement minoritaires, on calcule en fonction de ladite première valeur d'absorbance et des coefficients d'absorption desdits premiers dérivés chlorés, des premières valeurs absolues des concentrations des différents dérivés chlorés de l'acide isocyanurique, correspondant sensiblement aux valeurs absolues réelles des concentrations des différents dérivés chlorés de l'acide isocyanurique en équilibre dans ladite solution aqueuse.

De façon particulièrement avantageuse, le procédé de mesure conforme à l'invention comprend en outre les étapes suivantes : on détermine la valeur des concentrations des composés chlorés susceptibles d'être présents dans ladite solution ; on détermine les coefficients d'absorption desdits composés chlorés de façon à fournir, en fonction de ladite valeur des concentrations des composés chlorés, la contribution desdits composés chlorés à ladite première valeur de l'absorbance ; on déduit ladite contribution, de ladite première valeur de l'absorbance pour fournir une deuxième valeur de l'absorbance ; et, on détermine en fonction de ladite deuxième valeur de l'absorbance, desdits coefficients d'absorption desdits premiers dérivés chlorés de l'acide isocyanurique et desdites valeurs relatives des concentrations des différents dérivés chlorés de l'acide isocyanurique, des deuxièmes valeurs absolues des concentrations des différents dérivés chlorés de l'acide isocyanurique, lesdites deuxièmes valeurs absolues étant respectivement comprises entre lesdites premières valeurs absolues et lesdites valeurs absolues réelles.

Ainsi, selon cette caractéristique de l'invention, on augmente encore la précision des mesures de concentrations, en retranchant la contribution des composés chlorés tels que les chloramines ou l'acide hypochloreux, par exemple, qui absorbent le rayonnement électromagnétique entre 210 et 220 nanomètres, à la première valeur de l'absorbance de façon à obtenir une deuxième valeur de l'absorbance, plus représentative de la concentration en dérivés chlorés de l'acide isocyanurique.

Préférentiellement, on mesure ladite première valeur de l'absorbance dudit premier faisceau de rayonnement traversant ladite solution aqueuse, ledit premier faisceau de rayonnement ayant une longueur d'onde de 214, plus ou moins 2, nanomètres. Ainsi, l'atténuation dudit premier faisceau de rayonnement étant maximum dans cet intervalle de longueurs d'onde pour les formes ionisées H₂Cy⁻ et HClCy⁻, on obtient une sensibilité maximale de détection de ces dérivés chlorés.

Selon un mode de mise en oeuvre de l'invention particulièrement avantageux, le procédé de mesure comprend en outre les étapes suivantes : on mesure l'absorbance d'un second faisceau de rayonnement traversant ladite solution aqueuse, ledit second faisceau de rayonnement ayant une longueur d'onde non comprise entre 210 et 220 nanomètres et située en dehors des longueurs d'onde pour lesquelles les dérivés chlorés de l'acide isocyanurique sont susceptibles d'absorber, de façon à déterminer l'absorbance parasite des autres composés de ladite solution aqueuse ; et, on déduit ladite mesure de l'absorbance dudit second faisceau de ladite mesure de l'absorbance dudit premier faisceau de façon à fournir une mesure de l'absorbance dudit premier faisceau correspondant essentiellement à l'absorbance totale desdits premiers dérivés chlorés de l'acide isocyanurique. Ainsi, selon cette caractéristique de mise en oeuvre, on améliore encore la mesure des valeurs absolues des concentrations des différents dérivés chlorés de l'acide isocyanurique en déduisant de la mesure de la valeur de l'absorbance du premier faisceau de rayonnement l'absorbance parasite due aux autres composés de la solution aqueuse. De la sorte, on obtient des troisièmes valeurs absolues des concentrations des dérivés chlorés de l'acide isocyanurique et plus précisément des formes ionisées H₂Cy⁻ et HClCy⁻, comprises entre les valeurs réelles et lesdites deuxièmes valeurs absolues.

De façon avantageuse, on mesure l'absorbance d'un second faisceau de rayonnement d'une longueur d'onde de 235 nanomètres pour déterminer la valeur de l'absorbance parasite.

Un second objet de la présente invention est de proposer un dispositif de mesure de la concentration des dérivés chlorés de l'acide isocyanurique en équilibre dans une solution aqueuse selon la revendication 6.

Ainsi, lesdits deuxièmes et troisièmes moyens qui comprennent, respectivement, essentiellement une source d'excitation ultraviolette et par exemple un photomultiplicateur, comme on l'expliquera plus en détail dans la suite de la description, permettent de déterminer au moins l'absorbance totale de premiers dérivés chlorés de l'acide isocyanurique absorbant entre 210 et 220 nanomètres. En outre, les premiers et quatrièmes moyens comprennent essentiellement des moyens de calcul permettant d'une part, grâce à la mesure de l'acidité, de fournir les valeurs relatives des concentrations des dérivés chlorés de l'acide isocyanurique en fonction les unes des autres, et d'autre part, de fournir les valeurs absolues des concentrations des dérivés chlorés de l'acide isocyanurique, en fonction desdites valeurs relatives, de ladite absorbance totale et des coefficients d'absorption de chacun desdits premiers dérivés chlorés de l'acide isocyanurique.

Le dispositif comprend en outre, des moyens pour mesurer le pH de ladite solution aqueuse de façon à fournir sa concentration en ions H₃O⁺ représentative de l'acidité. Ainsi, l'acidité de la solution aqueuse qui est susceptible de fluctuer de façon importante dans le temps est mesurée en continu de manière à pouvoir calculer simultanément les valeurs relatives des concentrations des dérivés chlorés, les unes par rapport aux autres.

Pour ce faire, lesdits premiers moyens comportent des moyens pour calculer lesdites valeurs relatives en fonction des constantes d'équilibre entre les différents dérivés chlorés de l'acide isocyanurique et de ladite acidité de ladite solution aqueuse. Ces moyens pour calculer lesdites valeurs relatives sont essentiellement constitués d'une unité centrale comportant un microprocesseur, comme on l'expliquera de façon plus détaillée dans la suite de la description et sont mis en oeuvre par un premier programme de calcul.

Préférentiellement, lesdits quatrièmes moyens comportent des moyens pour pondérer les concentrations de chacune des concentrations desdits premiers dérivés chlorés de l'acide isocyanurique contribuant à ladite première valeur de l'absorbance dudit premier faisceau par lesdits coefficients d'absorption et pour calculer, au moyen desdites valeurs relatives des concentrations des différents dérivés chlorés de l'acide isocyanurique, lesdites premières valeurs absolues. Ces moyens pour pondérer et pour calculer sont constitués également de ladite unité centrale comportant ledit microprocesseur et sont mis en oeuvre par un deuxième programme de calcul.

Avantageusement, le dispositif comprend en outre, des moyens pour mesurer la concentration en composés chlorés de ladite solution aqueuse, de façon à déduire leur contribution à la première valeur de l'absorbance en fonction de leur coefficient d'absorption pour obtenir une deuxième valeur de l'absorbance.

De manière particulièrement avantageuse, selon un mode de mise en oeuvre de l'invention, le dispositif comprend : des moyens pour déterminer, en fonction de ladite concentration en composés chlorés et des coefficients d'absorption desdits composés chlorés, la contribution desdits composés chlorés à ladite première valeur de l'absorbance ; des moyens pour déduire ladite contribution, de ladite première valeur de l'absorbance et pour fournir une deuxième valeur de l'absorbance ; et, des moyens pour déterminer en fonction de ladite deuxième valeur de l'absorbance, desdits coefficients d'absorption desdits premiers dérivés chlorés de l'acide isocyanurique et desdites valeurs relatives, des deuxièmes valeurs absolues des concentrations des différents dérivés chlorés de l'acide isocyanurique, lesdites deuxièmes valeurs absolues étant respectivement comprises entre lesdites premières valeurs absolues et lesdites valeurs absolues réelles. Préférentiellement, ces moyens sont essentiellement constitués par ladite unité centrale comportant le microprocesseur et sont mis en oeuvre par au moins un troisième programme de calcul.

Selon une caractéristique préférentielle, les deuxièmes moyens pour produire au moins un premier faisceau de rayonnement, sont susceptibles de produire un second faisceau de rayonnement de longueur d'onde non comprise entre 210 et 220 nanomètres et situé en dehors des longueurs d'onde pour lesquelles les dérivés chlorés de l'acide isocyanurique sont susceptibles d'absorber, de façon à déterminer l'absorbance parasite des autres composés de ladite solution aqueuse et en ce que lesdits troisièmes moyens pour mesurer l'absorbance dudit premier faisceau de rayonnement sont susceptibles de mesurer l'absorbance dudit second faisceau de façon à déduire ladite mesure de l'absorbance dudit second faisceau de ladite mesure de l'absorbance dudit premier faisceau pour fournir une mesure de l'absorbance dudit premier faisceau correspondant essentiellement à l'absorbance total desdits premiers dérivés chlorés de l'acide isocyanurique. Ainsi, les deuxièmes moyens constitués d'une source d'excitation ultraviolette dont la gamme spectrale est relativement large, produit un second faisceau de rayonnement dont la longueur d'onde se situe en dehors des bandes d'absorption des dérivés chlorés de l'acide isocyanurique, par exemple 235 nanomètres. De la sorte, lesdits troisièmes moyens permettent de mesurer l'absorbance dudit second faisceau pour en déduire l'absorbance parasite dudit premier faisceau.

Selon une caractéristique de mise en oeuvre de l'invention particulièrement avantageuse, lesdits deuxièmes moyens pour produire au moins un premier faisceau de rayonnement comprennent également une fibre optique, raccordée à ladite source pour orienter lesdits faisceaux de rayonnement vers ladite solution aqueuse. Ainsi, le faisceau de rayonnement est dirigé vers la solution aqueuse qu'il traverse au moins partiellement, et il est susceptible d'être analysé après avoir traversé la solution. Préférentiellement, ladite fibre optique présente une partie centrale dans laquelle s'achemine le faisceau incident et une partie périphérique a travers lequel le faisceau réfléchi est susceptible de s'acheminer après avoir traversé ladite solution afin d'être orienté vers lesdits troisièmes moyens pour mesurer l'absorbance dudit premier faisceau de rayonnement.

Avantageusement, lesdits troisièmes moyens pour mesurer l'absorbance dudit premier faisceau de rayonnement comprennent un détecteur susceptible de mesurer l'intensité d'un faisceau de rayonnement reçu. Et, de préférence, ladite source d'excitation électromagnétique est constituée d'une lampe flash au xénon.

Selon encore un autre objet, la présente invention propose un ensemble de mesure de la concentration des dérivés chlorés de l'acide isocyanurique en solution aqueuse, comprenant un dispositif selon l'invention inséré dans un des deux compartiments d'un boîtier, ladite fibre optique se prolongeant dans l'autre compartiment dans lequel se situe la solution aqueuse à analyser, le faisceau de rayonnement émis dans ladite fibre optique y étant réfléchi au moyen d'un miroir disposé à une distance déterminée et en regard de son extrémité de façon à réorienter le faisceau dont l'intensité est susceptible d'être atténué par les composés de la solution qu'il traverse vers ledit détecteur.

De la sorte, l'ensemble de mesure est implantable au bord d'une piscine, par exemple, de façon que l'eau de ladite piscine puisse circuler en continu dans ledit autre compartiment et qu'elle puisse être analysée en continue dans le premier compartiment. Comme on l'expliquera dans la description détaillée qui va suivre, ladite unité centrale est munie d'un périphérique de sortie de type écran ou cadran indicateur permettant de lire directement la mesure de la concentration des dérivés chlorés de l'acide isocyanurique présents dans ladite solution aqueuse et par exemple l'eau de piscine.

D'autres particularités et avantages de l'invention ressortiront à la lecture de la description faite ci-après de modes de réalisation particuliers de l'invention, donnés à titre indicatif mais non limitatif, en référence aux dessins annexés sur lesquels :
- la Figure 1 est une vue schématique de la première partie d'un ensemble comportant un dispositif susceptible de mettre en oeuvre le procédé conforme à l'invention ; et,
- la Figure 2, est une vue schématique de la seconde partie de l'ensemble représenté sur la Figure 1.

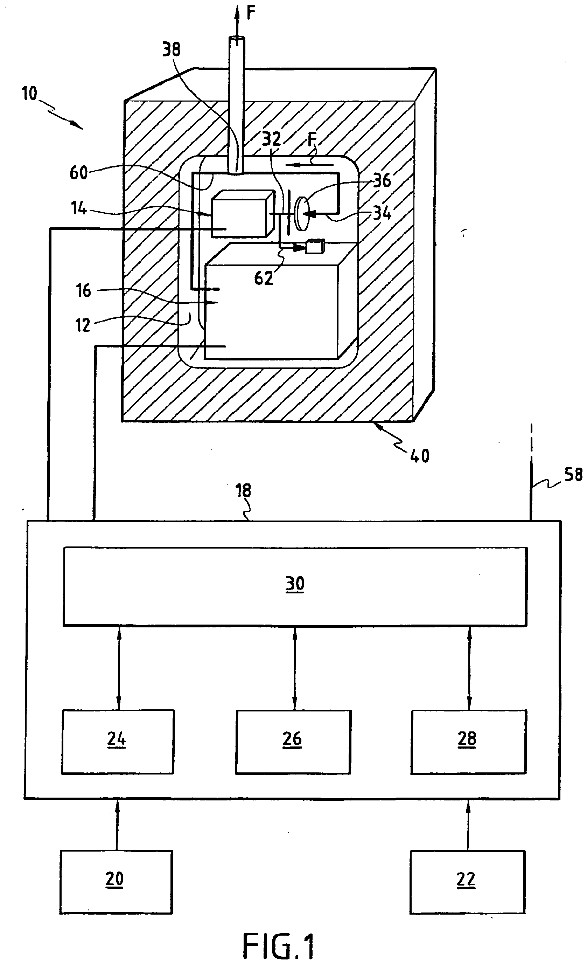

On se référera à la Figure 1 pour décrire les éléments constitutifs du dispositif permettant la mise en oeuvre du procédé conforme à l'invention, lequel dispositif est inséré dans un ensemble de mesure.

L'ensemble comporte une première partie 10 dans laquelle est ménagé un premier compartiment 12, lequel contient des moyens 14 pour produire un faisceau de rayonnement et des moyens 16 pour mesurer l'intensité d'un signal électromagnétique. En outre, ces moyens 14 et 16 sont raccordés à des moyens de traitement et de contrôle constitués d'une unité centrale 18 laquelle est munie d'un périphérique de contrôle 20, par exemple de type clavier, et d'un périphérique de sortie 22, par exemple de type écran. L'unité centrale comporte un microprocesseur 24, une mémoire 26 et un périphérique d'entrée 28, par exemple du type disque dur, le tout couplé à un bus 30.

Les moyens 14 pour produire un faisceau de rayonnement d'une longueur d'onde située dans le domaine de l'ultraviolet sont formés, de façon préférentielle, d'une lampe flash miniature au xénon qui permet, grâce à un encombrement minimal, 2cm de diamètre sur 2cm de long, de constituer un dispositif extrêmement compact. Ce type de source d'excitation ultraviolette présente une gamme spectrale située entre 190 et 1100 nanomètres et sa puissance moyenne est de 20 Watts. En outre, la fréquence de répétition des flashs s'étend de 100 à 300 Hertz. La lampe flash au xénon est susceptible d'être commandée par lesdits moyens de traitement et de contrôle, notamment pour modifier la fréquence d'émission du faisceau de rayonnement. Le faisceau de rayonnement émet, selon la présente invention, à deux fréquences particulières, 214 nanomètres plus ou moins deux nanomètres et 235 nanomètres.

D'autres sources d'excitation sont bien évidemment utilisables, par exemple les lasers accordables dans l'ultraviolet.

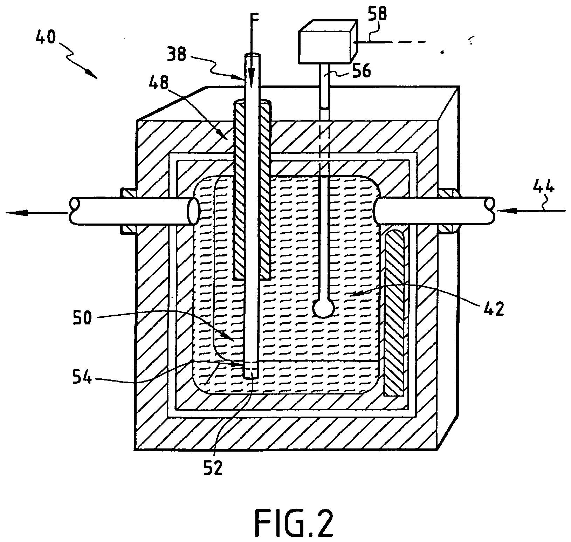

Lesdits moyens 14 sont susceptibles de produire un faisceau de rayonnement 32 focalisé dans une première portion de fibre optique 34 au moyen d'une lentille convergente 36. La première portion de fibre optique 34 est raccordée à une deuxième portion de fibre optique 38 qui se prolonge dans une seconde partie 40 de l'ensemble de mesure, que l'on décrira en référence à la Figure 2, et qui présente, une fibre centrale susceptible de guider l'onde électromagnétique incidente provenant de la première portion de fibre optique 34 dans le sens de la flèche F et des fibres périphériques susceptibles de guider une onde réfléchie dans le sens opposé.

La Figure 2 illustre la seconde partie 40 dans laquelle est ménagé un second compartiment 42 étanche, lequel présente une entrée 44 et une sortie 46, ainsi qu'un orifice 48 par lequel la seconde portion de fibre optique 38 est enfilée pour déboucher à l'intérieur du compartiment 42. Le second compartiment est susceptible d'être rempli d'une solution aqueuse, constituée par exemple d'eau de piscine, laquelle pénètre par l'entrée 44 et s'évacue par la sortie 46.

L'extrémité 50 de la seconde portion de fibre optique 38, comporte un miroir optique 52 disposé au regard de l'extrémité 50 à une distance déterminée de façon à ménager un espace 54 que la solution aqueuse occupe lorsqu'elle présente dans le second compartiment 42. De la sorte, le faisceau de rayonnement incident 32 qui se propage dans la fibre centrale de la deuxième portion de fibre optique 38 selon F, est susceptible, à son extrémité 50, de traverser l'espace 54 contenant la solution aqueuse, de se réfléchir sur le miroir optique 52 et de retraverser la solution pour emprunter le chemin optique défini par les fibres périphériques de la deuxième portion de fibre optique 38 dans le sens inverse à la propagation du rayon incident 32.

En outre, la seconde partie 40 de l'ensemble de mesure comporte des moyens 56 du type pH-mètre pour mesurer le pH de la solution aqueuse présente dans le second compartiment 42. Ces moyens 56 sont raccordés par des moyens de connexion 58 à l'unité centrale 18 illustrée sur la Figure 1 à laquelle ils délivrent directement sous forme numérique les valeurs de pH de la solution aqueuse.

On se référera de nouveau à la Figure 1 pour décrire des moyens aptes à mesurer la valeur de l'absorption du faisceau de rayonnement incident 32 par les constituants de la solution aqueuse que l'on décrira plus en détail dans la suite de la description. Le faisceau de rayonnement étant réfléchi par le miroir 52, le faisceau réfléchi se propage, dans le sens opposé à F, à travers la deuxième portion de fibre optique 38 dans les fibres périphériques, pour se propager ensuite dans une troisième portion de fibre optique 60.

La troisième portion de fibre optique 60 est raccordée aux moyens 16 pour mesurer l'intensité d'un signal électromagnétique. Ces moyens 16 sont constitués de moyens de détection du type tube photomultiplicateur miniature mais ils sont susceptibles d'être remplacés par une photodiode ou un spectrographe miniature à barrettes CCD. Ils permettent d'amplifier l'intensité d'un signal photonique s'acheminant dans la troisième portion de fibre 60, et de le transformer en un signal électrique dont la valeur est représentative de ladite intensité du signal photonique.

En outre, le faisceau de rayonnement incident 32 issu des moyens 14, c'est à dire de la lampe flash, est dérivé vers les moyens 16 pour mesurer l'intensité d'un signal électromagnétique, grâce à des moyens de dérivation 62 de façon à comparer l'intensité du signal incident et du signal réfléchi provenant de la troisième portion de fibre optique 60 et donner une mesure de l'absorption due à la solution aqueuse. Les moyens 16 comportent, en outre, des moyens permettant de collecter les données des mesures des signaux électromagnétiques et des moyens pour transmettre à l'unité centrale 18 des valeurs sous forme numérique directement représentative de la valeur de l'absorbance.

Ainsi, les moyens de traitement et de contrôle sont susceptibles, d'une part de commander le fonctionnement des moyens 14 pour produire un faisceau de rayonnement, des moyens 16 pour mesurer l'intensité d'un signal électromagnétique et du pH-mètre, d'autre part de traiter les valeurs des absorbances et les valeurs de pH mesurées.

De la sorte, l'ensemble de mesure ci-dessus décrit est adapté pour mesurer la concentration de dérivés chlorés de l'acide isocyanurique contenus dans une solution aqueuse, par exemple de l'eau de piscine, traversant le second compartiment. Cette mesure étant susceptible d'être effectuée en temps réel ou de manière séquentielle.

En préalable au démarrage de l'ensemble de mesure, il est nécessaire d'enregistrer un certain nombre de données dans la mémoire 26 de l'unité centrale 18 ; une partie de ces données portant sur des valeurs d'absorbance, est susceptible d'être déterminée par le dispositif de mesure et collectée directement. Pour ce faire, des solutions aqueuses étalons sont introduites dans le second compartiment 42, et on mesure l'absorbance de chacune des solutions étalon.

D'autres données nécessaires au fonctionnement de l'ensemble de mesure sont constituées, des coefficients d'absorption de chacun des dérivés chlorés de l'acide isocyanurique susceptibles d'être présents dans la solution, des constantes d'équilibre, c'est à dire des constantes d'ionisation et d'hydrolyse de ces dérivés dans l'eau, et éventuellement des coefficients d'absorption des composés chlorés susceptibles d'être présents dans la solution, si l'ensemble de mesure est muni de moyens aptes à mesurer leurs concentrations. Ces composés chlorés comprennent notamment l'acide hypochloreux, les chloramines et autres matières organiques chlorées.

En outre, le périphérique d'entrée 28 comprend les différents programmes de calcul, dans un langage de programmation usuel, par exemple en C, permettant de traiter les données et les valeurs des mesures. Ainsi, il comprend un premier programme de calcul destiné à calculer les valeurs relatives des différents dérivés chlorés de l'acide isocyanurique à partir des constantes d'équilibre et d'ionisation et des valeurs des mesures de pH données par le pH-mètre 56. Les eaux de piscine, dans lesquelles on introduit des dichloroisocyanurates de sodium ou de potassium et dont le pH est situé généralement entre 6,5 et 7,8 présentent essentiellement les ions isocyanuriques H₂Cy⁻ et chloroisocyanurique HClCy⁻. De la sorte, les valeurs relatives de la concentration de ces deux espèces sont données par une première équation à deux inconnues.

Le périphérique d'entrée 28 comprend un deuxième programme permettant d'affecter les coefficients d'absorption des dérivés chlorés à leur valeur de concentration, pour en faire respectivement le produit, la somme desdits produits correspondant à la valeur de l'absorbance qui est donnée par lesdits moyens de détection 16. Dans le cas des eaux de piscine décrites ci-dessus, lesquelles comprennent essentiellement les ions isocyanurique H₂Cy⁻ et chloroisocyanurique HClCy⁻, seule leur contribution est prise en compte dans la valeur de l'absorbance, selon une première aproximation. Ainsi, le deuxième programme permet de former une deuxième équation avec lesdites deux inconnues et de déterminer la valeur absolue desdites concentrations en ions isocyanurique H₂Cy⁻ et chloroisocyanurique HClCy⁻ lesquels sont les dérivés chlorés majoritaires de l'acide isocyanuriques présents dans la solution.

Cependant, d'autres espèces identifiées sont susceptibles de contribuer, dans une moindre proportion, à la valeur de l'absorbance totale mesurée, c'est notamment le cas des composés chlorés. Ainsi, avantageusement l'ensemble de mesure comprend des moyens de mesure desdits composés chlorés, non représentés, susceptibles de fournir une valeur de leur concentration. Le périphérique d'entrée 28 comprend alors un troisième programme permettant de calculer la valeur de la contribution des composés chlorés à la valeur de l'absorbance totale et ainsi d'obtenir une valeur de l'absorbance correspondant de façon plus précise à la contribution des espèces majoritaires H₂Cy⁻ et HClCCy⁻. Cette nouvelle valeur de l'absorbance est susceptible d'être introduite dans le deuxième programme de calcul pour affiner le calcul des valeurs absolues des concentrations en ions isocyanurique H₂Cy⁻ et chloroisocyanurique HClCy⁻.

Toutefois, des espèces chimiques non identifiées contribuent à la valeur de l'absorbance totale, et elles sont susceptibles d'être déduites de cette valeur de l'absorbance totale en mesurant la valeur de l'absorbance d'un second faisceau de rayonnement d'une longueur d'onde de 235 nanomètres où les dérivés chlorés de l'acide isocyanurique n'absorbent pas. Bien évidemment, une autre fréquence pourrait être choisie, par exemple 265 nanomètres, puisque la source d'excitation le permet. De la sorte, grâce à un quatrième programme de calcul, le microprocesseur 24 est susceptible de déduire cette valeur d'absorbance à 235 nanomètre de la valeur de l'absorbance totale à 214 nanomètre de façon à fournir une mesure encore plus précise de l'absorbance due aux ions isocyanurique H₂Cy⁻ et chloroisocyanurique HClCCy⁻.

Bien évidemment, grâce aux périphériques de contrôle 20 et de sortie 22, les programmes de calcul et les données de la mémoire 26 sont susceptibles d'être modifiés en fonction de la nature de la solution aqueuse à traiter.

Un avantage de la présente invention est de pouvoir suivre en temps réel sur le périphérique de sortie 22, la concentration en dérivés chlorés de l'acide isocyanurique de façon séquentielle ou en continu, et avec une précision bien supérieure aux méthodes de l'art antérieur de la technique.

## Revendications

1. Procédé de mesure de la concentration des dérivés chlorés de l'acide isocyanurique en équilibre dans une solution aqueuse, **caractérisé en ce qu'**il comprend les étapes suivantes :
- on détermine les coefficients d'absorption de chacun des premiers dérivés chlorés de l'acide isocyanurique dans ladite solution aqueuse susceptibles d'absorber un faisceau de rayonnement d'une longueur d'onde comprise entre 210 et 220 nanomètres ;
- on détermine l'acidité de ladite solution aqueuse pour fournir des valeurs relatives des concentrations des différents dérivés chlorés de l'acide isocyanurique en équilibre dans ladite solution aqueuse ;
- on mesure une première valeur de l'absorbance d'un premier faisceau de rayonnement traversant ladite solution aqueuse, ledit premier faisceau de rayonnement ayant une longueur d'onde comprise entre 210 et 220 nanomètres, de façon à déterminer au moins la valeur de l'absorbance totale de premiers dérivés chlorés de l'acide isocyanurique ; et,
- on détermine en fonction de ladite première valeur de l'absorbance dudit premier faisceau de rayonnement traversant ladite solution aqueuse, desdits coefficients d'absorption desdits premiers dérivés chlorés de l'acide isocyanurique et desdites valeurs relatives des concentrations des différents dérivés chlorés de l'acide isocyanurique, des premières valeurs absolues des concentrations des différents dérivés chlorés de l'acide isocyanurique, lesdites premières valeurs absolues correspondant sensiblement aux valeurs absolues réelles des concentrations des différents dérivés chlorés de l'acide isocyanurique en équilibre dans ladite solution aqueuse.

2. Procédé de mesure de la concentration des dérivés chlorés de l'acide isocyanurique selon la revendication 1, **caractérisé en ce qu'**il comprend en outre les étapes suivantes :
- on détermine la valeur des concentrations des composés chlorés susceptibles d'être présents dans ladite solution ;
- on détermine les coefficients d'absorption desdits composés chlorés de façon à fournir, en fonction de ladite valeur des concentrations des composés chlorés, la contribution desdits composés chlorés à ladite première valeur de l'absorbance ;
- on déduit ladite contribution, de ladite première valeur de l'absorbance pour fournir une deuxième valeur de l'absorbance ; et,
- on détermine en fonction de ladite deuxième valeur de l'absorbance, desdits coefficients d'absorption desdits premiers dérivés chlorés de l'acide isocyanurique et desdites valeurs relatives des concentrations des différents dérivés chlorés de l'acide isocyanurique, des deuxièmes valeurs absolues des concentrations des différents dérivés chlorés de l'acide isocyanurique, lesdites deuxièmes valeurs absolues étant respectivement comprises entre lesdites premières valeurs absolues et lesdites valeurs absolues réelles.

3. Procédé de mesure de la concentration des dérivés chlorés de l'acide isocyanurique selon la revendication 1 ou 2, **caractérisé en ce qu'**on mesure ladite première valeur de l'absorbance dudit premier faisceau de rayonnement traversant ladite solution aqueuse, ledit premier faisceau de rayonnement ayant une longueur d'onde de 214, plus ou moins 2, nanomètres.

4. Procédé de mesure de la concentration des dérivés chlorés de l'acide isocyanurique selon l'une quelconque des revendications 1 à 3, **caractérisé en ce qu'**il comprend en outre les étapes suivantes :
- on mesure l'absorbance d'un second faisceau de rayonnement traversant ladite solution aqueuse, ledit second faisceau de rayonnement ayant une longueur d'onde non comprise entre 210 et 220 nanomètres et située en dehors des longueurs d'onde pour lesquelles les dérivés chlorés de l'acide isocyanurique sont susceptibles d'absorber, de façon à déterminer l'absorbance parasite des autres composés de ladite solution aqueuse ; et,
- on déduit ladite mesure de l'absorbance dudit second faisceau de ladite mesure de l'absorbance dudit premier faisceau de façon à fournir une mesure de l'absorbance dudit premier faisceau correspondant essentiellement à l'absorbance totale desdits premiers dérivés chlorés de l'acide isocyanurique.

5. Procédé de mesure de la concentration des dérivés chlorés de l'acide isocyanurique selon la revendication 4, **caractérisé en ce qu'**on mesure l'absorbance d'un second faisceau de rayonnement d'une longueur d'onde de 235 nanomètres.

6. Dispositif de mesure de la concentration des dérivés chlorés de l'acide isocyanurique en équilibre dans une solution aqueuse, le dispositif comprenant :
- des moyens (56) pour mesurer le pH de ladite solution aqueuse de façon à fournir une mesure de l'acidité ;
- des premiers moyens (18,20, 22,24, 26,28, 30) comprenant un premier programme de calcul destiné à calculer à partir de l'acidité de ladite solution aqueuse, des valeurs relatives des concentrations des différents dérivés chlorés de l'acide isocyanurique, lesdites valeurs relatives correspondant sensiblement aux valeurs relatives réelles des concentrations des différents dérivés chlorés de l'acide isocyanurique en équilibre dans ladite solution aqueuse ;
Le dispositif de mesure étant **caractérisé en ce qu'**il comprend également :
- des deuxièmes moyens (14) comprenant une source d'excitation ultraviolette pour produire au moins un premier faisceau de rayonnement (32) d'une longueur d'onde comprise entre 210 et 220 nanomètres, susceptibles de traverser ladite solution aqueuse ;
- des troisièmes moyens (16) de détection pour mesurer une première valeur de l'absorbance dudit premier faisceau de rayonnement traversant ladite solution aqueuse, de façon à déterminer au moins l'absorbance totale de premiers dérivés chlorés de l'acide isocyanurique susceptibles d'absorber un faisceau de rayonnement d'une longueur d'onde comprise entre 210 et 220 nanomètres ; et,
- des quatrièmes moyens (18,20, 22,24, 26,28, 30) comprenant un deuxième programme de calcul pour déterminer en fonction de ladite première valeur de l'absorbance dudit premier faisceau de rayonnement traversant ladite solution aqueuse, des coefficients d'absorption de chacun desdits premiers dérivés chlorés de l'acide isocyanurique, et desdites valeurs relatives des concentrations des différents dérivés chlorés de l'acide isocyanurique, des premières valeurs absolues des concentrations des différents dérivés chlorés de l'acide isocyanurique, lesdites premières valeurs absolues correspondant sensiblement aux valeurs absolues réelles des concentrations des différents dérivés chlorés de l'acide isocyanurique en équilibre dans ladite solution aqueuse.

7. Dispositif de mesure de la concentration des dérivés chlorés de l'acide isocyanurique selon la revendication 6, **caractérisé en ce que** lesdits premiers moyens (18,20, 22,24, 26,28, 30) comportent des moyens pour calculer lesdites valeurs relatives en fonction des constantes d'équilibre entre les différents dérivés chlorés de l'acide isocyanurique et de ladite acidité de ladite solution aqueuse.

8. Dispositif de mesure de la concentration des dérivés chlorés de l'acide isocyanurique selon la revendication 6 ou 7, **caractérisé en ce que** lesdits quatrièmes moyens (18,20, 22,24, 26,28, 30) comportent des moyens pour pondérer les concentrations de chacune des concentrations desdits premiers dérivés chlorés de l'acide isocyanurique contribuant à ladite première valeur de l'absorbance dudit premier faisceau par lesdits coefficients d'absorption et pour calculer, au moyen desdites valeurs relatives des concentrations des différents dérivés chlorés de l'acide isocyanurique, lesdites premières valeurs absolues.

9. Dispositif de mesure de la concentration des dérivés chlorés de l'acide isocyanurique selon l'une quelconque des revendications 6 à 8, **caractérisé en ce qu'**il comprend en outre, des moyens pour mesurer la concentration en composés chlorés de ladite solution aqueuse.

10. Dispositif de mesure de la concentration des dérivés chlorés de l'acide isocyanurique selon la revendication 9, **caractérisé en ce qu'**il comprend en outre :
- des moyens (18,20, 22,24, 26,28, 30) pour déterminer, en fonction de ladite concentration en composés chlorés et des coefficients d'absorption desdits composés chlorés, la contribution desdits composés chlorés à ladite première valeur de l'absorbance ;
- des moyens (18,20, 22,24, 26,28, 30) pour déduire ladite contribution, de ladite première valeur de l'absorbance et pour fournir une deuxième valeur de l'absorbance ; et,
- des moyens (18,20, 22,24, 26,28, 30) pour déterminer en fonction de ladite deuxième valeur de l'absorbance, desdits coefficients d'absorption desdits premiers dérivés chlorés de l'acide isocyanurique et desdites valeurs relatives, des deuxièmes valeurs absolues des concentrations des différents dérivés chlorés de l'acide isocyanurique, lesdites deuxièmes valeurs absolues étant respectivement comprises entre lesdites premières valeurs absolues et lesdites valeurs absolues réelles.

11. Dispositif de mesure de la concentration des dérivés chlorés de l'acide isocyanurique selon l'une quelconque des revendication 6 à 10, **caractérisé en ce que** les deuxièmes moyens (14) pour produire au moins un premier faisceau de rayonnement, sont susceptibles de produire un second faisceau de rayonnement de longueur d'onde non comprise entre 210 et 220 nanomètres et situé en dehors des longueurs d'onde pour lesquelles les dérivés chlorés de l'acide isocyanurique sont susceptibles d'absorber, de façon à déterminer l'absorbance parasite des autres composés de ladite solution aqueuse et **en ce que** lesdits troisièmes moyens pour mesurer l'absorbance dudit premier faisceau de rayonnement sont susceptibles de mesurer l'absorbance dudit second faisceau de façon à déduire ladite mesure de l'absorbance dudit second faisceau de ladite mesure de l'absorbance dudit premier faisceau pour fournir une mesure de l'absorbance dudit premier faisceau correspondant essentiellement à l'absorbance totale desdits premiers dérivés chlorés de l'acide isocyanurique.

12. Dispositif de mesure de la concentration des dérivés chlorés de l'acide isocyanurique selon l'une quelconque des revendications 6 à 11, **caractérisé en ce que** lesdits deuxièmes moyens (14) pour produire au moins un premier faisceau de rayonnement comprennent également une fibre optique (34,38), raccordée à ladite source pour orienter lesdits faisceaux de rayonnement vers ladite solution aqueuse.

13. Dispositif de mesure de la concentration des dérivés chlorés de l'acide isocyanurique selon l'une quelconque des revendications 6 à 12, **caractérisé en ce que** lesdits troisièmes moyens (16) pour mesurer l'absorbance dudit premier faisceau de rayonnement comprennent un détecteur susceptible de mesurer l'intensité d'un faisceau de rayonnement reçu.

14. Dispositif de mesure de la concentration des dérivés chlorés de l'acide isocyanurique selon la revendication 12, **caractérisé en ce que** ladite source d'excitation électromagnétique est constituée d'une lampe flash au xénon.

15. Ensemble de mesure de la concentration des dérivés chlorés de l'acide isocyanurique en solution aqueuse, **caractérisé en ce qu'**il comprend un dispositif selon les revendications 12 ou 14 et 13 inséré dans un des deux compartiments d'un boîtier, ladite fibre optique se prolongeant dans l'autre compartiment dans lequel se situe la solution aqueuse à analyser, le faisceau de rayonnement émis dans ladite fibre optique y étant réfléchi au moyen d'un miroir disposé à une distance déterminée et en regard de son extrémité de façon à réorienter le faisceau dont l'intensité est susceptible d'être atténué par les composés de la solution qu'il traverse vers ledit détecteur.

## Patentansprüche

1. Verfahren zur Messung der Konzentration chlorierter Isocyanursäurederivate im Gleichgewicht in einer wässrigen Lösung, **dadurch gekennzeichnet, dass** es die folgenden Schritte aufweist:
- Bestimmen der Absorptionskoeffizienten von jedem der ersten chlorierten Isocyanursäurederivate in der wässrigen Lösung, die geeignet sind, ein Strahlenbündel mit einer Wellenlänge zwischen 210 und 220 Nanometern zu absorbieren,
- Bestimmen des Säuregehalts der wässrigen Lösung, um relative Werte der Konzentrationen der verschiedenen chlorierten Isocyanursäurederivate im Gleichgewicht in der wässrigen Lösung bereitzustellen,
- Messen eines ersten Wertes der Absorption eines ersten Strahlenbündels, das die wässrige Lösung durchquert, wobei das erste Strahlenbündel eine Wellenlänge zwischen 210 und 220 Nanometern aufweist, derart, um mindestens den Wert der Gesamtabsorption von ersten Isocyanursäurederivaten zu bestimmen, und
- Bestimmen in Abhängigkeit von dem ersten Wert der Absorption des ersten Strahlenbündels, das die wässrige Lösung durchquert, den Absorptionskoeffizienten der ersten Isocyanursäurederivate und den relativen Werte der Konzentrationen der verschiedenen Isocyanursäurederivate, der ersten absoluten Werte der Konzentrationen der verschiedenen chlorierten Isocyanursäurederivate, wobei die ersten absoluten Werte im Wesentlichen den tatsächlichen absoluten Werten der Konzentrationen der verschiedenen chlorierten Isocyanursäurederivate im Gleichgewicht in der wässrigen Lösung entsprechen.

2. Verfahren zur Messung der Konzentration chlorierter Isocyanursäurederivate nach Anspruch 1, **dadurch gekennzeichnet, dass** es ferner die folgenden Schritte aufweist:
- Bestimmen der Werte der Konzentrationen der chlorierten Verbindungen, die in der wässrigen Lösung vorhanden sein können,
- Bestimmen der Absorptionskoeffizienten der chlorierten Verbindungen, derart, um in Abhängigkeit von dem Wert der Konzentrationen der chlorierten Verbindungen den Beitrag der chlorierten Verbindungen zu dem ersten Wert der Absorption bereitzustellen,
- Ableiten des Beitrags von dem ersten Wert der Absorption, um einen zweiten Wert der Absorption bereitzustellen, und
- Bestimmen in Abhängigkeit von dem zweiten Wert der Absorption, den Absorptionskoeffizienten der ersten chlorierten Isocyanursäurederivate und den relativen Werten der Konzentrationen der verschiedenen chlorierten Isocyanursäurederivate der zweiten absoluten Werte der Konzentrationen der verschiedenen chlorieren Isocyanursäurederivate, wobei die zweiten absoluten Werte jeweils zwischen den ersten absoluten Werten und den tatsächlichen absoluten Werten liegen.

3. Verfahren zur Messung der Konzentration chlorierter Isocyanursäurederivate nach Anspruch 1 oder 2, **dadurch gekennzeichnet, dass** der erste Wert der Absorption des ersten Strahlenbündels, das die wässrige Lösung durchquert, gemessen wird, wobei das Strahlenbündel eine Wellenlänge von 214 plus minus 2 Nanometern aufweist.

4. Verfahren zur Messung der Konzentration chlorierter Isocyanursäurederivate nach einem der Ansprüche 1 bis 3, **dadurch gekennzeichnet, dass** es ferner die folgenden Schritte aufweist:
- Messen der Absorption eines zweiten Strahlenbündels, das die wässrige Lösung durchquert, wobei das zweite Strahlenbündel eine Wellenlänge außerhalb des Bereichs zwischen 210 und 220 Nanometern aufweist und sich außerhalb der Wellenlängen befindet, für die die Isocyanursäurederivate absorbieren können, derart, um die parasitäre Absorption der anderen Verbindungen der wässrigen Lösung zu bestimmen, und
- Ableiten der Messung der Absorption des zweiten Strahlenbündels von der Messung der Absorption des ersten Strahlenbündels derart, um eine Messung der Absorption des ersten Strahlenbündels bereitzustellen, die im Wesentlichen der Gesamtabsorption der ersten Isocyanursäurederivate entspricht.

5. Verfahren zur Messung der Konzentration chlorierter Isocyanursäurederivate nach Anspruch 4, **dadurch gekennzeichnet, dass** die Absorption eines zweiten Strahlenbündels von einer Wellenlänge von 235 Nanometern gemessen wird.

6. Vorrichtung zur Messung der Konzentration chlorierter Isocyanursäurederivate im Gleichgewicht in einer wässrigen Lösung, wobei die Vorrichtung aufweist:
- Mittel (56) zum Messen des pH-Wertes der wässrigen Lösung, derart, um eine Messung des Säuregehalts bereitzustellen,
- erste Mittel (18, 20, 22, 24, 26, 28, 30), umfassend ein erstes Rechenprogramm, das dazu bestimmt ist, aus dem Säuregehalt der wässrigen Lösung relative Werte der Konzentrationen der verschiedenen chlorierten Isocyanursäurederivate zu berechnen, wobei die relativen Werte im Wesentlichen den tatsächlichen relativen Werten der Konzentrationen der verschiedenen chlorierten Isocyanursäurederivate im Gleichgewicht in der wässrigen Lösung entsprechen,
wobei die Vorrichtung zur Messung **dadurch gekennzeichnet ist, dass** sie auch aufweist:
- zweite Mittel (14), umfassend eine UV-Erregerquelle, um mindestens ein erstes Strahlenbündel (32) von einer Wellenlänge zwischen 210 und 220 Nanometern zu erzeugen, die geeignet sind, die erste wässrige Lösung zu durchqueren,
- dritte Mittel (16) zum Erfassen, um einen ersten Wert der Absorption des ersten Strahlenbündels, das die wässrige Lösung durchquert, zu messen, derart, um mindestens die Gesamtabsorption von ersten chlorierten Isocyanursäurederivaten zu bestimmen, die geeignet sind, ein Strahlenbündel mit einer Wellenlänge zwischen 210 und 220 Nanometern zu absorbieren, und
- vierte Mittel (18, 20, 22, 24, 26, 28, 30), umfassend ein zweites Rechenprogramm, um in Abhängigkeit von dem ersten Wert der Absorption des ersten Strahlenbündels, das die wässrige Lösung durchquert, den Absorptionskoeffizienten der ersten chlorierten Isocyanursäurederivate und den relativen Werte der Konzentrationen der verschiedenen chlorierten Isocyanursäurederivate, erste absolute Werte der Konzentrationen der verschiedenen chlorierten Isocyanursäurederivate zu bestimmen, wobei die ersten absoluten Werte im Wesentlichen den tatsächlichen absoluten Werten der Konzentrationen der verschiedenen chlorierten Isocyanursäurederivate im Gleichgewicht in der wässrigen Lösung entsprechen.

7. Vorrichtung zur Messung der Konzentration chlorierter Isocyanursäurederivate nach Anspruch 6, **dadurch gekennzeichnet, dass** die ersten Mittel (18, 20, 22, 24, 26, 28, 30) Mittel aufweisen, um die relativen Werte in Abhängigkeit von den Gleichgewichtskonstanten zwischen den chlorierten Isocyanursäurederivaten und dem Säuregehalt der wässrigen Lösung zu berechnen.

8. Vorrichtung zur Messung der Konzentration chlorierter Isocyanursäurederivate nach Anspruch 6 oder 7, **dadurch gekennzeichnet, dass** die vierten Mittel (18, 20, 22, 24, 26, 28, 30) Mittel aufweisen, um die Konzentration von jeder der Konzentrationen der ersten chlorierten Isocyanursäurederivate, die zu dem ersten Wert der Absorption des ersten Strahlenbündels beitragen, durch die Absorptionskoeffizienten abzuwägen und um mittels der relativen Werte der Konzentrationen der verschiedenen chlorierten Isocyanursäurederivate die ersten absoluten Werte zu berechnen.

9. Vorrichtung zur Messung der Konzentration chlorierter Isocyanursäurederivate nach einem der Ansprüche 6 bis 8, **dadurch gekennzeichnet, dass** sie ferner Mittel zum Messen der Konzentration an chlorierten Verbindungen der wässrigen Lösung aufweist.

10. Vorrichtung zur Messung der Konzentration chlorierter Isocyanursäurederivate nach Anspruch 9, **dadurch gekennzeichnet, dass** sie ferner aufweist:
- Mittel (18, 20, 22, 24, 26, 28, 30) zum Bestimmen in Abhängigkeit von der Konzentrationen an chlorierten Verbindungen und der Absorptionskoeffizienten der chlorierten Verbindungen des Beitrags der chlorierten Verbindungen zu dem ersten Wert der Absorption,
- Mittel (18, 20, 22, 24, 26, 28, 30) zum Ableiten des Beitrags von dem ersten Wert der Absorption und zum Bereitstellen eines zweiten Wertes der Absorption und
- Mittel (18, 20, 22, 24, 26, 28, 30) zum Bestimmen in Abhängigkeit von dem zweiten Wert der Absorption, den Absorptionskoeffizienten der ersten chlorierten Isocyanursäurederivate und den relativen Werten der zweiten absoluten Werte der Konzentrationen der verschiedenen chlorieren Isocyanursäurederivate, wobei die zweiten absoluten Werte jeweils zwischen den ersten absoluten Werten und den tatsächlichen absoluten Werten liegen.

11. Vorrichtung zur Messung der Konzentration chlorierter Isocyanursäurederivate nach einem der Ansprüche 6 bis 10, **dadurch gekennzeichnet, dass** die zweiten Mittel (14) zum Erzeugen von mindestens einem ersten Strahlenbündel geeignet sind, ein zweites Strahlenbündel von einer Wellenlänge außerhalb des Bereichs zwischen 210 und 220 Nanometern und die sich außerhalb der Wellenlängen befindet, für die die Isocyanursäurederivate absorbieren können, derart zu erzeugen, um die parasitäre Absorption der anderen Verbindungen der wässrigen Lösung zu bestimmen, und dass die dritten Mittel zum Messen der Absorption des ersten Strahlenbündels geeignet sind, die Absorption des zweiten Strahlenbündels derart zu messen, um die Messung der Absorption des zweiten Strahlenbündels von der Messung der Absorption des ersten Strahlenbündels abzuleiten, um eine Messung der Absorption des ersten Strahlenbündels bereitzustellen, die im Wesentlichen der Gesamtabsorption der ersten chlorierten Isocyanursäurederivate entspricht.

12. Vorrichtung zur Messung der Konzentration chlorierter Isocyanursäurederivate nach einem der Ansprüche 6 bis 11, **dadurch gekennzeichnet, dass** die zweiten Mittel (14) zum Erzeugen von mindestens einem ersten Strahlenbündel auch eine Glasfaser (34, 38) aufweisen, die mit der Quelle verbunden ist, um die Strahlenbündel in Richtung der wässrigen Lösung auszurichten.

13. Vorrichtung zur Messung der Konzentration chlorierter Isocyanursäurederivate nach einem der Ansprüche 6 bis 12, **dadurch gekennzeichnet, dass** die dritten Mittel (16) zum Messen der Absorption des ersten Strahlenbündels einen Detektor aufweisen, der geeignet ist, die Intensität eines empfangenen Strahlenbündels zu messen.

14. Vorrichtung zur Messung der Konzentration chlorierter Isocyanursäurederivate nach Anspruch 12, **dadurch gekennzeichnet, dass** die elektromagnetische Erregungsquelle aus einer Xenonblitzlampe besteht.

15. Anordnung zur Messung der Konzentration chlorierter Isocyanursäurederivate in wässriger Lösung, **dadurch gekennzeichnet, dass** sie eine Vorrichtung nach den Ansprüchen 12 oder 14 und 13 aufweist, die in einer der zwei Kammern eines Gehäuses eingefügt ist, wobei sich die Glasfaser in der anderen Kammer erstreckt, in der sich die zu analysierende wässrige Lösung befindet, wobei das Strahlenbündel, das in die Glasfaser emittiert wird, darin durch einen Spiegel reflektiert wird, der in einem bestimmten Abstand und gegenüber von ihrem Ende derart angeordnet ist, um das Bündel umzulenken, dessen Intensität geeignet ist, durch die Verbindungen der Lösung, die es durchquert, in Richtung des Detektors abgeschwächt zu werden.

## Claims

1. Method for measuring the concentration of chlorinated derivatives of isocyanuric acid in equilibrium in an aqueous solution, **characterized in that** it comprises the following steps:
- the absorption coefficients of each of the first chlorinated derivatives of isocyanuric acid in the aqueous solution, which are capable of absorbing a radiation beam with a wavelength between 210 and 220 nanometers, are determined;
- the acidity of the aqueous solution is determined so as to provide relative values of the concentrations of the various chlorinated derivatives of isocyanuric acid in equilibrium in the aqueous solution;
- a first value of the absorbance of a first radiation beam passing through the aqueous solution is measured, wherein the first radiation beam has a wavelength of between 210 and 220 nanometers, in order to determine at least the value of total absorbance of the first chlorinated derivatives of isocyanuric acid; and,
- according to the first value of the absorbance of the first radiation beam passing through the aqueous solution, to said absorption coefficients of said first chlorinated derivatives of isocyanuric acid and to said relative values of the concentrations of the various chlorinated derivatives of the isocyanuric acid, the first absolute values of the concentrations of the various chlorinated derivatives of isocyanuric acid are determined, said first absolute values substantially corresponding to the actual absolute values of the concentrations of the various chlorinated derivatives of isocyanuric acid in equilibrium in said aqueous solution.

2. Method for measuring the concentration of chlorinated derivatives of isocyanuric acid according to claim 1, **characterized in that** it further comprises the following steps:
- the value of the concentrations of the chlorinated compounds that may be present in the solution is determined;
- the absorption coefficients of the chlorinated compounds are determined so as to provide, as a function of said value of the concentrations of the chlorinated compounds, the contribution of said chlorinated compounds to said first absorbance value;
- said contribution is determined based on said first absorbance value so as to provide a second absorbance value; and,
- as a function of said second absorbance value, of said absorption coefficients of said first chlorinated derivatives of isocyanuric acid and of the relative values of the concentrations of the various chlorinated derivatives of isocyanuric acid, the second absolute values of the concentrations of the various chlorinated derivatives of isocyanuric acid are determined, wherein said second absolute values are respectively between the first absolute values and the actual absolute values.

3. Method for measuring the concentration of chlorinated derivatives of isocyanuric acid according to claim 1 or 2, **characterized in that** the first absorbance value of the first radiation beam passing through the aqueous solution is measured, wherein the first radiation beam has a wavelength of 214 plus or minus 2 nanometers.

4. Method for measuring the concentration of chlorinated derivatives of isocyanuric acid according to any one of the claims 1 to 3, **characterized in that** it further comprises the following steps:
- the absorbance of a second radiation beam passing through the aqueous solution is measured, wherein the second radiation beam has a wavelength not comprised between 210 and 220 nm, and situated outside the wavelengths that the chlorinated derivatives of isocyanuric acid are capable of absorbing, in order to determine the parasitic absorbance of the other compounds of the aqueous solution; and,
- the measurement of the absorbance of the second beam is deduced from the measurement of the absorbance of the first beam so as to provide a measurement of the absorbance of the first beam substantially corresponding to the total absorbance of the first chlorinated derivatives of the isocyanuric acid.

5. Method for measuring the concentration of chlorinated derivatives of isocyanuric acid according to claim 4, **characterized in that** the absorbance of a second radiation beam of a wavelength of 235 nm is measured.

6. Device for measuring the concentration of chlorinated derivatives of isocyanuric acid in equilibrium in an aqueous solution, wherein the device comprises:
- means (56) for measuring the pH of the aqueous solution in order to provide a measurement of the acidity;
- first means (18, 20, 22, 24, 26, 28, 30) comprising a first calculation program for calculating from the acidity of the aqueous solution, relative values of the concentrations of the various chlorinated derivatives of the isocyanuric acid, wherein said relative values substantially correspond to the actual relative values of the concentrations of the various chlorinated derivatives of isocyanuric acid in equilibrium in the aqueous solution;
The measuring device being **characterized in that** it also comprises:
- second means (14) comprising an ultraviolet excitation source for producing at least a first radiation beam (32) with a wavelength between 210 and 220 nm, and that is capable of passing through the aqueous solution;
- third detection means (16) for measuring a first value of the absorbance of the first radiation beam passing through the aqueous solution, in order to determine at least the total absorbance of first chlorinated derivatives of isocyanuric acid that are capable of absorbing a radiation beam with a wavelength between 210 and 220 nm; and,
- fourth means (18, 20, 22, 24, 26, 28, 30) comprising a second calculation program for determining, as a function of said first value of the absorbance of said first radiation beam passing through the aqueous solution, of the absorption coefficients of each of said first chlorinated derivatives of isocyanuric acid, and of the relative values of the concentrations of the various chlorinated derivatives of isocyanuric acid, said first absolute values of the concentrations of the various chlorinated derivatives of isocyanuric acid, said first absolute values substantially corresponding to the actual absolute values of the concentrations of the various chlorinated derivatives of isocyanuric acid in equilibrium in the aqueous solution.

7. Device for measuring the concentration of chlorinated derivatives of isocyanuric acid according to claim 6, **characterized in that** said first means (18,20, 22,24, 26,28, 30) comprise means for calculating said relative values as a function of the equilibrium constants between the various chlorinated derivatives of isocyanuric acid and of said acidity of the aqueous solution.

8. Device for measuring the concentration of chlorinated derivatives of isocyanuric acid according to claim 6 or 7, **characterized in that** the fourth means (18, 20, 22, 24, 26, 28, 30) comprise means for weighting the concentrations of each of the concentrations of the first chlorinated derivatives of isocyanuric acid contributing to said first absorbance value of the first beam by said absorption coefficients, and for calculating said first absolute values by means of said relative values of the concentrations of the various chlorinated derivatives of the isocyanuric acid.

9. Device for measuring the concentration of chlorinated derivatives of isocyanuric acid according to any one of the claims 6 to 8, **characterized in that** it further comprises means for measuring the concentration of chlorinated compounds of the aqueous solution.

10. Device for measuring the concentration of chlorinated derivatives of isocyanuric acid according to claim 9, **characterized in that** it further comprises:
- means (18, 20, 22, 24, 26, 28, 30) for determining, as a function of the concentration of chlorinated compounds and of the absorption coefficients of the chlorinated compounds, the contribution of the chlorinated compounds to the first absorbance value;
- means (18, 20, 22, 24, 26, 28, 30) for deducing said contribution from said first absorbance value and for providing a second absorbance value; and,
- means (18, 20, 22, 24, 26, 28, 30) for determining, as a function of the second absorbance value, of said the absorption coefficients of the first chlorinated derivatives of isocyanuric acid and of said relative values, the second absolute values of the concentrations of the various chlorinated derivatives of isocyanuric acid, wherein the second absolute values are comprised respectively between the first absolute values and the actual absolute values.

11. Device for measuring the concentration of chlorinated derivatives of isocyanuric acid according to any one of the claims 6 to 10, **characterized in that** the second means (14) for producing at least a first radiation beam, are capable of producing a second radiation beam with a wavelength not comprised between 210 and 220 nm and located outside the wavelengths at which the chlorinated derivatives of isocyanuric acid are capable of absorbing, in order to determine the parasitic absorbance of the other compounds of the aqueous solution, and **in that** the third means for measuring the absorbance of the first radiation beam are capable of measuring the absorbance of the second beam in order to deduce the measurement of the absorbance of the second beam from the measurement of the absorbance of the first beam in order to provide a measurement of the absorbance of the first beam that substantially corresponds to the total absorbance of the first chlorinated derivatives of the isocyanuric acid.

12. Device for measuring the concentration of chlorinated derivatives of isocyanuric acid according to any one of the claims 6 to 11, **characterized in that** the second means (14) for producing at least a first radiation beam also comprise an optical fiber (34,38) that is connected to the source for orienting the radiation beams towards the aqueous solution.

13. Device for measuring the concentration of chlorinated derivatives of isocyanuric acid according to any one of the claims 6 to 12, **characterized in that** the third means (16) for measuring the absorbance of the first radiation beam comprise a detector capable of measuring the intensity of a received radiation beam.

14. Device for measuring the concentration of chlorinated derivatives of isocyanuric acid according to claim 12, **characterized in that** the electromagnetic excitation source consists of a xenon flash lamp.

15. Measuring assembly for the concentration of chlorinated derivatives of isocyanuric acid in aqueous solution, **characterized in that** it comprises a device according to claims 12 or 14 and 13 inserted in one of the two compartments of a housing, wherein the optical fiber extends into the other compartment in which the aqueous solution to be analyzed is located, and wherein the radiation beam emitted in the optical fiber is reflected by means of a mirror arranged at a specified distance and facing its end in such a way as to reorient the beam whose intensity is likely to be attenuated by the compounds of the solution it passes through towards the detector.
